# FASCICULE DE BREVET EUROPEEN

(11) **EP 0 825 835 B1**
(45) Date de publication et mention de la délivrance du brevet: **22.08.2001**
(21) Numéro de dépôt: 96916185.0
(22) Date de dépôt: 10.05.1996
(51) Int. Cl.: A61B 17/68

(54) **DISPOSITIF D'OSTEOSYNTHESE IMPLANTABLE**
IMPLANTIERBARE OSTEOSYNTHESEVORRICHTUNG
IMPLANTABLE OSTEOSYNTHESIS DEVICE

(30) Priorité: 19.05.1995 FR 9506089
(43) Date de publication de la demande: 04.03.1998
(73) Titulaire: Klein, Jean-Michel, 31400 Toulouse (FR); Trouillet, Michel, 31600 Eaunes (FR)
(72) Inventeur: Klein, Jean-Michel, 31400 Toulouse (FR); Trouillet, Michel, 31600 Eaunes (FR)
(74) Mandataire: Cabinet BARRE LAFORGUE & associés
(86) Numéro de dépôt international: FR9600706
(87) Numéro de publication internationale: WO9636291

(56) Documents cités:
- EP-A- 0 301 898
- EP-A- 0 553 782
- GB-A- 2 208 476
- US-A- 4 279 248

## Description

L'invention concerne un dispositif d'ostéosynthèse implantable.

L'objectif essentiel visé par tout dispositif d'ostéosynthèse implantable consiste dans l'obtention d'un effet de bridage des parties osseuses apte à garantir une consolidation osseuse effective. A cet effet, tous les dispositifs d'ostéosynthèse actuels sont conçus pour présenter, une fois implantés, une solidité et une rigidité qui permettent de garantir l'obtention d'un parfait effet de bridage. Toutefois, il s'avère qu'afin de satisfaire à cet objectif, toutes les solutions proposées ont conduit à la conception de dispositifs d'ostéosynthèse dont la mise en place nécessite une technique opératoire longue et complexe et une instrumentation ancillaire conséquente.

Ainsi, par exemple, dans le cadre de l'ostéosynthèse du sternum notamment en vue de la fermeture d'une sternotomie, un matériel couramment utilisé consiste en des agrafes dites de COTREL-DUBOUSSET comportant un premier crochet fixé sur une tige filetée, un deuxième crochet destiné à coulisser dans le sens du rapprochement vers le crochet fixe, et des moyens de blocage en translation et en rotation de ce deuxième crochet comprenant un système écrou/contre-écrou, et une vis-pression introduite dans la base dudit deuxième crochet.

Bien que les expérimentations cliniques aient conclu à la fiabilité de ce matériel qui permet d'obtenir une bonne consolidation osseuse, la technique opératoire de mise en place de ce dernier s'avère par contre relativement complexe et délicate. En effet, cette mise en place impose d'utiliser une pince de rapprochement destinée à assurer le coulissement du crochet mobile jusqu'à mise en compression des crochets, puis tout en maintenant cette mise en compression, d'assurer le blocage du crochet mobile en premier lieu au moyen du système écrou/contre-écrou, et en second lieu au moyen de la vispression. Ensuite, l'excédent de tige doit être sectionné au moyen d'une pince coupante, et la tête de la vis-pression également sectionnée de façon à ne pas faire saillie.

Dans la pratique, il s'avère que la mise en place de deux ou trois agrafes, nécessaires pour l'ostéosynthèse du sternum, nécessite un laps de temps non négligeable de l'ordre d'un quart d'heure. De plus, il est à noter que de telles agrafes ne présentent pas une totale fiabilité du fait que la vis-pression ne saurait garantir de façon absolue un blocage en rotation du crochet mobile.

Un autre dispositif décrit dans EP-301 898 permet de pallier certains de ces inconvénients, mais s'avère inapte à solutionner l'ensemble de ces derniers.

La présente invention vise à pallier les inconvénients précités des dispositifs d'ostéosynthèse actuels et a pour principal objectif de fournir un dispositif d'ostéosynthèse, d'une part, dont la technique opératoire de mise en place est très simple et très aisée et nécessite une instrumentation ancillaire minimale, et d'autre part, permettant d'obtenir un parfait effet de bridage des parties osseuses.

Un autre objectif de l'invention est de fournir un dispositif d'ostéosynthèse compressive et distractive.

Un autre objectif de l'invention est de fournir un dispositif d'ostéosynthèse permettant de réaliser de façon très aisée une ostéotomie.

A cet effet, l'invention vise un dispositif d'ostéosynthèse implantable tel que défini dans le préambule de la revendication 1, et se caractérisant en ce que le système de cliquet comprend une dent asymétrique associée aux moyens élastiques de façon à pénétrer dans les crans de l'élément longiforme avec un jeu apte à permettre audit système de cliquet de basculer par rapport audit corps de façon à obtenir un effet de coin assurant un auto-blocage.

En premier lieu, la technique opératoire de mise en place d'un tel dispositif d'ostéosynthèse est très simple et rapide du fait que le blocage en translation des parties fixe et mobile est obtenu automatiquement et de façon instantanée lors du déplacement relatif desdites parties fixe et mobile en raison du caractère unidirectionnel des moyens de verrouillage incorporés dont sont dotés ces dernières.

De ce fait, cette mise en place nécessite simplement de déplacer relativement les parties fixe et mobile jusqu'à obtenir l'effet de bridage souhaité qui s'avère de surcroît irréversible et définitif, puis éventuellement de sectionner l'excédent de l'élément longiforme pour les applications où cet excédent peut être gênant.

Une telle conception conduit donc, d'une part, à un gain de temps notable par rapport aux techniques opératoires de mise en place des dispositifs d'ostéosynthèse connus, et d'autre part, à une simplification extrême de l'instrumentation ancillaire nécessaire.

De plus, un tel dispositif d'ostéosynthèse peut être utilisé en vue d'ostéosynthèses compressives ou distractives, selon le sens de déplacement relatif des parties fixe et mobile autorisé par les moyens de verrouillage.

En second lieu, la dent asymétrique des moyens de verrouillage unidirectionnel a pour seule fonction d'assurer le maintien en position de la partie mobile, les efforts sollicitant cette dernière tendant à conduire à un effet de coin qui entraîne l'auto-blocage de ladite partie mobile. De ce fait, la taille relativement réduite des moyens de verrouillage ne constitue pas une fragilisation du dispositif d'ostéosynthèse qui s'avère, grâce au phénomène d'auto-blocage, présenter une rigidité, une solidité et une fiabilité garantissant un parfait effet de bridage.

Selon une autre caractéristique de l'invention, la dent asymétrique s'étend en saillie par rapport à une des faces, dite supérieure, d'un pion d'encliquetage et comporte une face frontale inclinée apte à permettre le déplacement de la partie mobile selon un sens de déplacement, et une face frontale opposée de blocage en translation de ladite partie mobile, la portion de la face supérieure du pion d'encliquetage jouxtant la face inclinée de la dent asymétrique s'étendant dans un plan inférieur par rapport à la portion de ladite face supérieure jouxtant la face de blocage de ladite dent asymétrique.

Une telle conception permet en effet de contrôler de façon aisée le jeu de basculement conduisant à l'effet d'auto-blocage, de façon à obtenir cet effet tout en assurant le maintien en position de la dent asymétrique à l'intérieur du cran correspondant.

Par ailleurs, le corps de la partie mobile comporte avantageusement un logement débouchant radialement dans le manchon apte à loger le système de cliquet.

De plus, en vue de former le logement du système de cliquet, le corps de la partie mobile est préférentiellement percé d'un alésage ménagé transversalement par rapport au manchon à partir d'une des faces externes dudit corps, de façon à former, à l'opposé de ladite face externe par rapport audit manchon, ledit logement.

En outre, selon une autre caractéristique de l'invention :
- le corps de la partie mobile est percé d'un alésage ménagé transversalement par rapport au manchon à partir d'une des faces externes dudit corps, de façon à déboucher dans le fond du logement du système de cliquet, ledit alésage présentant une section inférieure à celle dudit logement,
- le système de cliquet comporte, à l'opposé de la dent asymétrique, un axe de dimensions adaptées pour s'étendre dans l'alésage et déboucher à l'extérieur du corps de la partie mobile, ledit axe étant doté, au niveau de son extrémité débouchante, d'un organe de manoeuvre apte à permettre de le faire pivoter sur lui-même.

Une telle disposition a pour avantage de permettre, au moyen d'un même dispositif, une ostéosynthèse compressive et/ou distractive en orientant de façon adéquate le système de cliquet par l'intermédiaire de l'organe de manoeuvre. De plus, elle permet de réaliser des réglages de la position relative de la partie mobile, ainsi que de procéder au démontage du dispositif.

En outre, cette disposition s'avère particulièrement avantageuse pour des interventions telles qu'ostéotomie, chirurgie du rachis, ... nécessitant d'écarter dans un premier temps deux parties osseuses en vue de la mise en place de greffons, puis de comprimer ces parties osseuses en vue de permettre l'ostéointégration. En effet, et tel qu'on le comprendra mieux plus loin, ces deux opérations peuvent être effectuées sans nécessiter d'instrumentation (davier, ...) telle qu'utilisée à l'heure actuelle, l'écartement puis la compression étant obtenus successivement au moyen du seul dispositif d'ostéosynthèse, avec inversion de la position du système de cliquet entre ces deux opérations.

Selon un premier mode de réalisation préférentiel de l'invention visant un dispositif d'ostéosynthèse particulièrement destiné à la consolidation de sternums, notamment après sternotomie, ou à la chirurgie du rachis cervical :
- la partie fixe comporte un élément longiforme constitué d'une tige rectiligne, et des moyens de liaison comprenant un corps solidaire d'une des extrémités de la tige rectiligne et un organe de liaison s'étendant dans le prolongement dudit corps et présentant la forme d'un crochet apte à venir coopérer avec un os,
- la partie mobile comprend un organe de liaison s'étendant dans le prolongement du corps de ladite partie mobile et présentant la forme d'un crochet similaire à celui de la partie fixe agencé pour s'étendre parallèlement à ce dernier.

De plus, de façon avantageuse, la tige rectiligne comporte alors longitudinalement au moins une face plane, le manchon ménagé dans le corps de la partie mobile présentant une section conjuguée de celle de ladite tige rectiligne.

Cette forme de la tige rectiligne garantit en effet un blocage en rotation relatif des parties fixe et mobile.

Selon un deuxième mode de réalisation préférentiel visant un dispositif d'ostéosynthèse particulièrement destiné à l'ostéosynthèse d'os longs tels que fémur, tibia, humérus, ... mais pouvant s'appliquer également à la consolidation de sternums:
- la partie fixe comporte un élément longiforme constitué d'une lame rigide rectiligne recourbée vers une de ses extrémités de façon à former un coude de 180 degrés apte à venir coopérer avec un os,
- la partie mobile comprend un organe de liaison porté par le corps de ladite partie mobile et constitué d'une lame rigide courbe formant un coude de 180 degrés conjugué de celui de la lame rigide de la partie fixe, agencé pour s'étendre parallèlement à ce dernier, ledit coude étant décalé latéralement par rapport à l'axe longitudinal de l'élément longiforme de la partie fixe.

Selon un troisième mode de réalisation préférentiel visant un dispositif d'ostéosynthèse particulièrement destiné à une ostéosynthèse trochantérienne ou olécranienne :
- la partie fixe comporte un élément longiforme constitué d'une broche cylindrique dotée d'une extrémité en forme de pointe et comprenant une rainure annulaire à proximité de ladite extrémité, et un organe de liaison présentant la forme d'une griffe dotée d'un corps comportant une gorge transversale apte à venir se loger dans la rainure annulaire de la broche,
- la partie mobile comprend un organe de liaison s'étendant dans le prolongement du corps de ladite partie mobile et présentant la forme d'une griffe similaire à celle de la partie fixe agencée pour s'étendre parallèlement à cette dernière.

Selon un autre mode de réalisation préférentiel visant un dispositif d'ostéosynthèse particulièrement destiné à la réalisation d'ostéotomies :
- la partie fixe comporte un élément longiforme constitué d'une lame rigide présentant deux tronçons d'extrémité rectilignes parallèles reliés par un tronçon médian incliné par rapport à l'axe longitudinal de ces derniers, un desdits tronçons d'extrémité présentant un orifice apte à loger une tête de vis, et l'autre tronçon d'extrémité étant doté de moyens de verrouillage,
- l'organe mobile comporte au moins un organe de liaison apte à pénétrer dans un os.

En outre, dans ce cas, lorsque le système de cliquet est associé à un organe de manoeuvre permettant de modifier son orientation, les opérations d'écartement puis de compression des parties osseuses peuvent être réalisées, après prédécoupe de l'os et mise en place de la vis et de l'organe de liaison, sans nécessiter d'instrumentation annexe.

Selon un autre mode de réalisation préférentiel visant un dispositif d'ostéosynthèse particulièrement destiné à la chirurgie rachidienne :
- la partie fixe comporte un élément longiforme constitué d'une tige rectiligne, et des moyens de liaison comprenant un corps monté sur ledit élément longiforme prolongé par un axe de section polygonale, et un organe de liaison consistant en une vis pédiculaire dotée d'une tête percée d'une lumière de section polygonale conjuguée de celle de l'axe du corps,
- la partie mobile comporte des moyens de liaison comprenant un axe de section polygonale s'étendant dans le prolongement du corps de ladite partie mobile, parallèlement par rapport à l'axe des moyens de liaison de la partie fixe, et un organe de liaison consistant en une vis pédiculaire dotée d'une tête percée d'une lumière de section polygonale conjuguée de celle dudit axe.

Un tel dispositif permet, à partir des points d'ancrage, de mettre chaque élément en situation de traction ou de distraction-compression autorisant ainsi, outre la réduction des déformations anatomiques, une mise en précontrainte du système d'ostéosynthèse qui augmente sa résistance mécanique face aux sollicitations auxquels sont soumis les segments vertébraux pathologiques.

De plus, la forme polygonale des axes et des lumières des têtes de vis offre une pluralité de possibilités de réglages en rotation (fonction du nombre de faces du polygone) qui autorise d'incliner la partie fixe dans le plan sagittal de façon à obtenir une réduction des déformations anatomiques du type lordose ou sciphose.

Selon une autre caractéristique de l'invention visant un dispositif d'ostéosynthèse destiné à la chirurgie rachidienne et permettant de réaliser un montage en cadre augmentant notablement la résistance d'ensemble dudit dispositif, celui-ci comprend deux dispositifs d'ostéosynthèse rachidienne tels que décrits ci-dessus disposés parallèlement, et un dispositif d'ostéosynthèse doté de crochets ou d'organes de liaison en forme de coude de 180 degrés, tel que décrit plus haut, disposé de façon à relier les éléments longiformes des deux dispositifs d'ostéosynthèse précités.

Selon une autre caractéristique de l'invention visant un dispositif d'ostéosynthèse destiné à la chirurgie rachidienne et permettant de réaliser un montage à plusieurs étages comportant trois points d'ancrage, ce dernier comprend :
- un ensemble fixe composé de deux parties fixes comportant chacune un élément longiforme constitué d'une tige rectiligne, et des moyens de liaison comprenant un corps monté sur ledit élément longiforme, vers une des extrémités de ce dernier, prolongé par un axe de section polygonale, et un organe de liaison consistant en une vis pédiculaire dotée d'une tête percée d'une lumière de section polygonale conjuguée de celle de l'axe du corps,
- un ensemble mobile intermédiaire de liaison des éléments longiformes de l'ensemble fixe au droit d'un tronçon de ces derniers opposé à l'extrémité portant les moyens de liaison, adapté pour que lesdits éléments longiformes s'étendent parallèlement en étant partiellement décalés longitudinalement, ledit ensemble mobile intermédiaire comprenant une partie mobile montée sur chaque élément longiforme, et des moyens de liaison comportant, d'une part, un axe de section polygonale reliant les corps desdites parties mobiles et s'étendant orthogonalement par rapport aux éléments longiformes et, d'autre part, un organe de liaison consistant en une vis pédiculaire dotée d'une tête présentant une gorge de section polygonale conjuguée de celle dudit axe et un filetage externe en partie supérieure, et en un capuchon apte à venir se visser sur la partie supérieure de ladite tête.

Par ailleurs, dans le cadre des applications de l'invention à la chirurgie rachidienne, le corps des moyens de liaison de chaque partie fixe peut avantageusement comporter des moyens de verrouillage unidirectionnel aptes à permettre de le déplacer dans un seul sens de déplacement relativement à l'élément longiforme correspondant.

D'autres caractéristiques, buts et avantages de l'invention ressortiront de la description détaillée qui suit en référence aux dessins annexés qui en représentent, à titre d'exemples non limitatifs, sept modes de réalisation préférentiels et une variante de réalisation. Sur ces dessins qui font partie intégrante de la présente description :
- la figure 1 est une vue en perspective d'un premier mode de réalisation d'un dispositif d'ostéosynthèse conforme à l'invention destiné à l'ostéosynthèse du sternum ou à la chirurgie du rachis cervical,
- la figure 2 en est une coupe longitudinale par un plan axial,
- la figure 3 en est une coupe longitudinale partielle à échelle agrandie représentant la partie mobile dans sa position d'auto-blocage,
- la figure 4 en est une coupe transversale, à échelle agrandie, par un plan A,
- la figure 5 est une vue en perspective d'un deuxième mode de réalisation d'un dispositif d'ostéosynthèse conforme à l'invention destiné à une ostéosynthèse trochantérienne ou olécranienne,
- la figure 6 en est une coupe longitudinale partielle par un plan B,
- la figure 7 est une vue en perspective d'un troisième mode de réalisation d'un dispositif d'ostéosynthèse conforme à l'invention destiné à une ostéosynthèse d'os longs ou du sternum,
- la figure 8 est une vue en perspective d'un quatrième mode de réalisation d'un dispositif d'ostéosynthèse conforme à l'invention destiné à la réalisation d'ostéotomies,
- la figure 9 est une vue en perspective d'un cinquième mode de réalisation d'un dispositif d'ostéosynthèse conforme à l'invention destiné à la chirurgie rachidienne,
- la figure 10 est une vue en perspective d'un sixième mode de réalisation d'un dispositif d'ostéosynthèse conforme à l'invention destiné également à la chirurgie rachidienne,
- la figure 11 est une vue de dessus partiellement en biais d'un septième mode de réalisation d'un dispositif d'ostéosynthèse conforme à l'invention également destiné à la chirurgie rachidienne,
- et la figure 12 est une coupe longitudinale partielle à échelle agrandie illustrant une variante de réalisation du système de cliquet d'un dispositif d'ostéosynthèse conforme à l'invention.

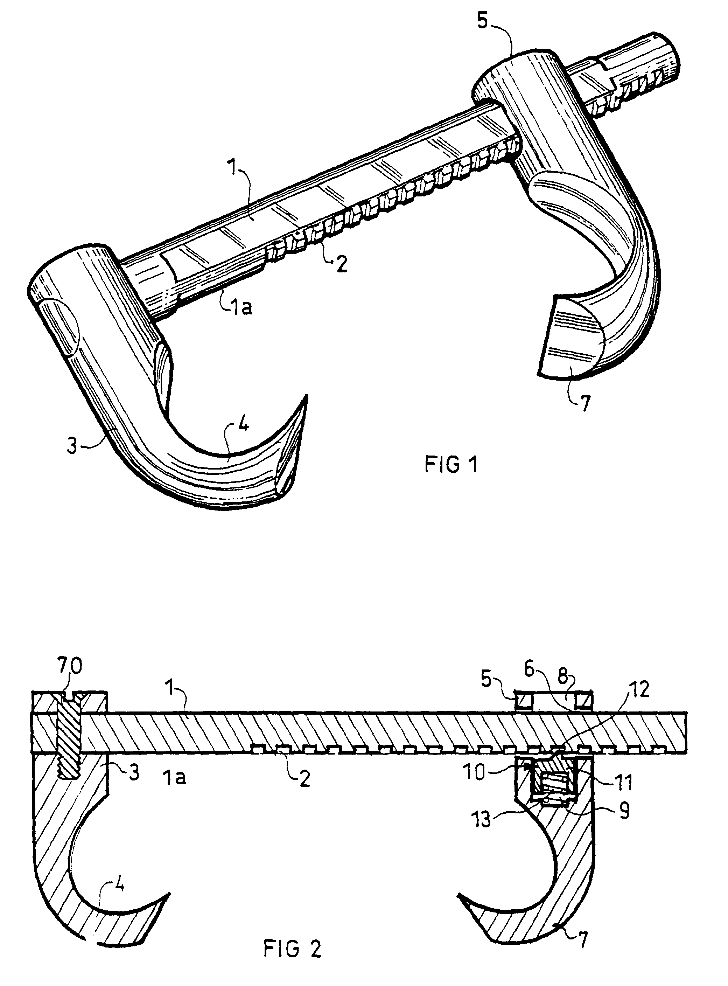

Les dispositifs d'ostéosynthèse représentés aux figures 1 à 11 comportent tous une partie dite fixe dotée de moyens de liaison avec un os, une partie dite mobile également dotée de moyens de liaison avec un os, ainsi que des moyens de verrouillage incorporés aptes à permettre le déplacement relatif desdites parties fixe et mobile dans un seul sens de déplacement, et à assurer leur auto-blocage selon l'autre sens de déplacement.

En premier lieu, le dispositif représenté aux figures 1 à 4 est particulièrement destiné à l'ostéosynthèse du sternum après sternotomie, ou à titre préventif dans le cas de déhiscences sternales, ainsi qu'à la chirurgie du rachis cervical.

La partie fixe de ce dispositif d'ostéosynthèse comprend une tige rigide 1 de section sensiblement carrée dont une des faces 1a, dite inférieure, comprend, à partir d'une des extrémités de ladite tige et sur sensiblement les trois-quart de sa longueur, des crans transversaux 2.

Cette partie fixe comprend, en outre, au niveau de son extrémité dépourvue de crans 2, des moyens de liaison comportant un corps cylindrique 3 percé d'un alésage radial, emmanché sur la tige 1, et, dans le prolongement longitudinal dudit corps cylindrique, un organe de liaison en forme de crochet 4.

Tel que représenté à la figure 2, la solidarisation de la tige 1 et des moyens de liaison est assurée au moyen d'une vis sans tête 70 introduite dans un alésage taraudé ménagé axialement dans le corps cylindrique 3 et transversalement dans ladite tige 1.

Un tel mode de solidarisation a pour avantage de permettre, en cas d'urgence, de séparer rapidement les moyens de liaison de la tige 1, et ainsi d'autoriser un retrait rapide du dispositif d'ostéosynthèse. De plus, il permet d'inverser l'orientation du crochet 4 selon l'utilisation envisagée.

Tel que représenté aux figures 1 à 4, ces moyens de liaison sont, en outre, disposés de façon que le crochet 4 s'étende dans le prolongement de la face crantée 1a de la tige 1.

La partie mobile de ce dispositif d'ostéosynthèse, destinée à se déplacer le long de la tige 1, présente une forme externe similaire à celle des moyens de liaison de la partie fixe, et comporte donc un corps cylindrique 5 doté d'un alésage radial 6 formant un manchon de section sensiblement supérieure à celle de la tige 1 adapté pour obtenir un jeu entre lesdits corps et tige, et un organe de liaison en forme de crochet 7 s'étendant dans le prolongement dudit corps.

Le corps 5 est, en outre, percé d'un alésage longitudinal 8 à partir de la face d'extrémité de ce corps 5 opposée au crochet 7, de façon à former, à l'opposé de ladite face d'extrémité par rapport à l'alésage radial 6, un logement cylindrique 9 débouchant radialement dans ledit alésage radial.

De plus, cette partie mobile comprend un système de cliquet agencé pour venir se loger dans le logement 9 du corps cylindrique 5, et adapté pour coopérer avec les crans 2 de la tige 1 de façon à former avec ces derniers des moyens de verrouillage unidirectionnel.

Ce système de cliquet comprend, en premier lieu, un pion creux 10 obturé par une paroi supérieure 11 sur laquelle fait saillie une dent asymétrique 12 présentant une face inclinée 12a formant une pente de l'ordre de 45 degrés par rapport à ladite paroi supérieure, et une face de blocage 12b orthogonale par rapport à cette paroi supérieure.

En outre, la portion 11a de cette paroi supérieure 11 jouxtant la face inclinée 12a de la dent asymétrique 12, s'étend dans un plan inférieur par rapport à celui de la portion 11b de ladite paroi supérieure jouxtant la face de blocage 12b de ladite dent asymétrique. A titre d'exemple, le dénivelé entre les portions de surface 11a, 11b est par exemple de l'ordre de deux à trois dixièmes de millimètres.

Le système de cliquet comprend, enfin, un ressort hélicoïdal 13 logé à l'intérieur du pion 10 de façon à prendre appui sous la face supérieure 11 de ce dernier et sur la paroi de fond du logement 9, et à solliciter ledit système de cliquet vers sa position de verrouillage.

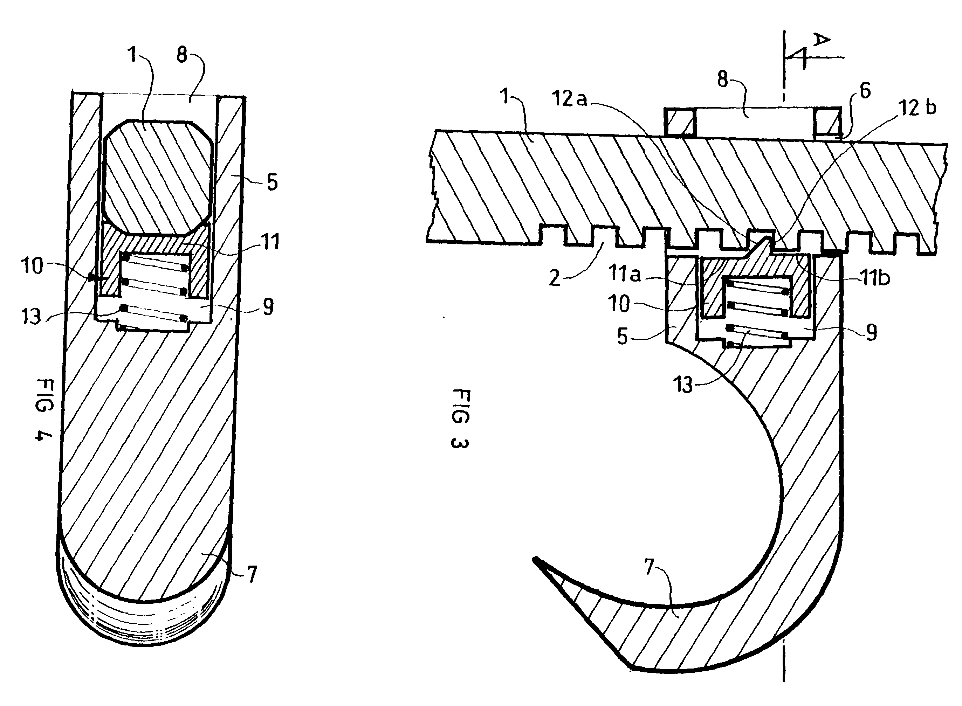

Tel que représenté à la figure 3, du fait du jeu de montage existant entre la tige 1 et le corps cylindrique, ce dernier est amené à pivoter dans sa position de verrouillage, ce pivotement étant en outre limité grâce au dénivelé de la face supérieure 11 du pion 10, conduisant à l'obtention d'un effet de coin et donc d'un auto-blocage lorsque la partie mobile est sollicitée par des efforts. De ce fait, les efforts ne sollicitent pas la dent asymétrique 12 qui a ainsi pour seule fonction d'assurer le maintien en position de la partie mobile.

Il est à noter que le dispositif représenté aux figures 1 à 4 est destiné à une ostéosynthèse compressive (verrouillage tendant à empêcher l'éloignement des crochets 4, 7). Toutefois, ce dispositif peut également être utilisé en vue d'ostéosynthèses distractives par simple inversion de la position de la partie mobile et des moyens de liaison.

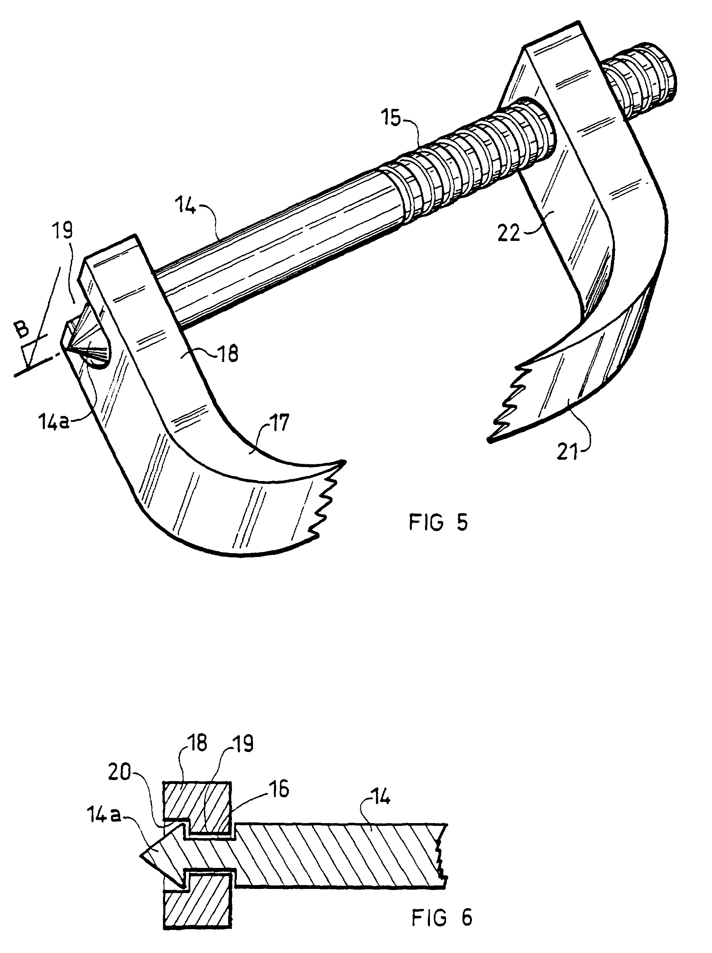

Le deuxième dispositif d'ostéosynthèse représenté aux figures 5, 6 est particulièrement destiné à une ostéosynthèse trochantérienne ou olécranienne.

La partie fixe de ce dispositif d'ostéosynthèse comprend une broche 14 dotée d'une extrémité en forme de pointe 14a, et présentant sur une portion de sa longueur à partir de son extrémité opposée, des crans annulaires 15.

En outre, cette broche 14 comprend une rainure annulaire 16 juxtaposée longitudinalement à son extrémité en forme de pointe 14a.

Cette partie fixe comprend, en outre, un organe de liaison présentant la forme d'une griffe 17 s'étendant dans le prolongement d'un corps 18 de forme parallélépipédique rectangle.

Afin d'assurer le positionnement relatif de cet organe de liaison et de la broche 14, ce dernier comporte un gorge transversale 19, ménagée dans la face d'extrémité du corps 18, apte à venir se loger dans la rainure annulaire 16 de ladite broche.

De plus, tel que représenté à la figure 6, cette gorge 19 présente longitudinalement un lamage 20 de section adaptée pour masquer partiellement la pointe 14a.

Compte-tenu de la faible longueur de cette pointe 14a, ce lamage 20 permet d'éviter, en masquant partiellement cette dernière, d'avoir à la sectionner en fin d'opération.

La partie mobile de ce dispositif d'ostéosynthèse présente une forme externe similaire à celle de l'organe de liaison 17, 18 de la partie fixe. Elle est donc constituée d'une griffe 21 venant dans le prolongement d'un corps 22 de forme parallélépipédique rectangle percé d'un alésage de section conjuguée de celle de la broche 14, ce corps 22 incorporant un système de cliquet identique à celui décrit en référence aux figures 1 à 4.

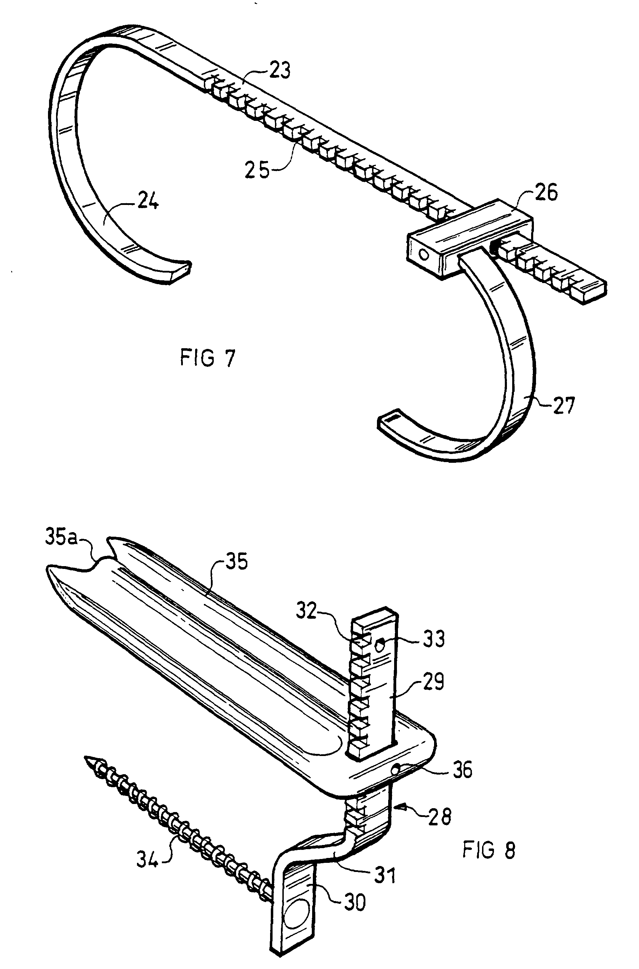

Le dispositif d'ostéosynthèse représenté à la figure 7 est particulièrement destiné à l'ostéosynthèse d'os longs (tibia, fémur, humérus, ...) ainsi qu'à l'ostéosynthèse du sternum.

La partie fixe de ce dispositif d'ostéosynthèse comprend une lame rigide rectiligne 23 de section rectangulaire (par exemple de 4 mm de largeur et 2 mm d'épaisseur) recourbée vers une de ses extrémités de façon à former un coude 24 de 180 degrés. A partir de son extrémité opposée et sur sensiblement les trois-quarts de sa longueur, cette lame rigide comporte une pluralité de crans 25 ménagés sur un des chants de cette dernière.

La partie mobile de ce dispositif d'ostéosynthèse comprend, quant à elle, un corps 26 présentant une forme parallélépipédique rectangle de faible épaisseur, percé transversalement d'un alésage rectangulaire de section conjuguée de celle de la lame rigide 23. Comme précédemment, ce corps 26 intègre un système de cliquet de verrouillage unidirectionnel des parties fixe et mobile.

Cette partie mobile comprend, en outre, un organe de liaison constitué d'une lame rigide courbe 27 formant un coude de 180 degrés dont une des extrémités est sertie ou soudée sur le corps 26 au droit d'une des faces latérales longitudinales de ce dernier.

Tel que représenté à la figure 7, les organes de liaison 24, 27 des parties fixe et mobile se trouvent ainsi décalés latéralement et permettent d'assurer un parfait bridage de l'os.

Le dispositif d'ostéosynthèse représenté à la figure 8 est particulièrement destiné à la réalisation d'ostéotomies.

La partie fixe de ce dispositif d'ostéosynthèse est constituée d'une lame rigide 28 de section rectangulaire et de forme adaptée pour épouser longitudinalement la forme d'une épiphyse. A cet effet, cette lame rigide 28 comporte deux tronçons d'extrémité 29, 30 rectilignes et parallèles reliés par un tronçon médian 31 incliné par rapport à l'axe longitudinal de ces derniers.

En outre, le tronçon d'extrémité supérieur 29 destiné à se trouver en regard d'une épiphyse, comporte un chant sur lequel sont ménagés une pluralité de crans 32. Ce tronçon supérieur 29 est également percé d'un orifice 33 ménagé à proximité de son extrémité supérieure destiné à permettre d'introduire une broche de guidage.

Le tronçon inférieur est, quant à lui, percé d'un orifice apte à loger la tête d'une vis 34.

L'organe mobile de ce dispositif d'ostéosynthèse comporte, quant à lui, une plaque 35 de conception classique en soi dotée d'une arête d'extrémité 35a tranchante. Vers son extrémité opposée, cette plaque 35 comporte une lumière transversale, adaptée pour permettre de la monter sur la partie fixe, en regard de laquelle est incorporé un système de cliquet conforme à l'invention.

Cette plaque 35 comporte, enfin, un alésage longitudinal traversant 36 ménagé de façon à permettre le guidage de cette dernière au moyen d'une broche de guidage, lors de son implantation.

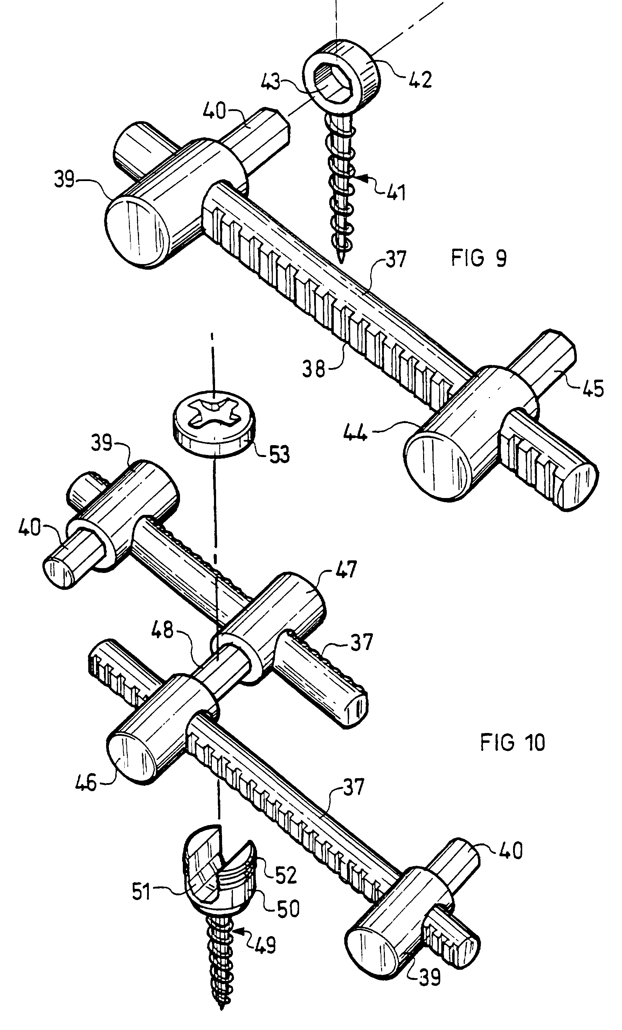

Les dispositifs d'ostéosynthèse représentés aux figures 9, 10, 11 sont tous trois destinés à la chirurgie rachidienne.

En premier lieu, le dispositif d'ostéosynthèse représenté à la figure 9 est conçu pour permettre un montage à un étage comportant deux points d'ancrage.

La partie fixe de ce dispositif d'ostéosynthèse comprend une tige rectiligne 37 dotée d'une face longitudinale plane dans laquelle sont ménagés une pluralité de crans 38.

Cette partie fixe comporte, en outre, des moyens de liaison comprenant un corps cylindrique 39 percé d'un alésage radial de section conjuguée de la tige 37 apte à permettre de le monter sur cette dernière, et dans le prolongement axial dudit corps, un axe 40 de section polygonale.

Tel que représenté à la figure 9, ces moyens de liaison sont conçus pour être associés à une vis pédiculaire d'ancrage 41 dotée d'une tête 42 percée d'une lumière 43 de section polygonale conjuguée de celle de l'axe 40. A titre d'exemple, cette lumière 43, et donc l'axe 40, peut être octogonale ou former une empreinte connue sous la dénomination "TORQ".

En outre, les moyens de liaison précités peuvent soit être fixes relativement à la tige 37, soit être équipés d'un système de cliquet conforme à l'invention autorisant leur translation dans un seul sens de déplacement.

La partie mobile de ce dispositif d'ostéosynthèse présente, quant à elle, une même structure que celle des moyens de liaison de la partie fixe et comprend donc un corps cylindrique 44 incorporant un système de cliquet, et un axe 45 de section polygonale apte à coopérer avec une vis pédiculaire telle que décrite ci-dessus.

Outre les avantages inhérents à la conception des dispositifs d'ostéosynthèse selon l'invention, un tel dispositif d'ostéosynthèse a pour intérêt d'offrir une pluralité de réglages en rotation dans le plan sagittal.

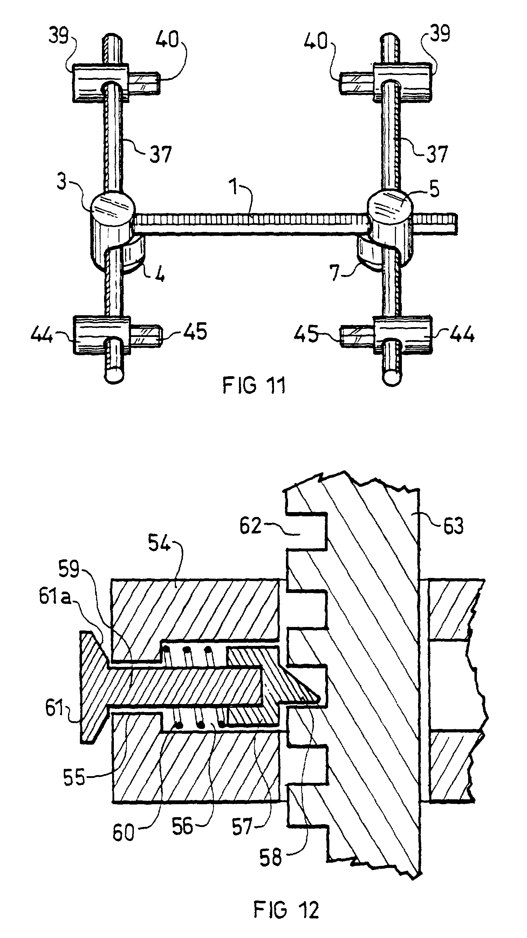

Le dispositif d'ostéosynthèse représenté à la figure 10 présente une même conception de base que celui décrit ci-dessus, mais il est conçu pour permettre un montage à plusieurs étages avec trois points d'ancrage. (A des fins de simplification, les mêmes références numériques seront donc utilisées pour désigner des éléments similaires.)

Ce dispositif d'ostéosynthèse comporte un ensemble fixe constitué de deux tiges 37 associées chacune à des moyens de liaison composés d'un corps cylindrique 39 et d'un axe polygonal 40, lesdites tiges étant disposées parallèlement, en position inversée l'une par rapport à l'autre, et décalées longitudinalement.

La partie mobile comprend, quant à elle, deux corps cylindriques 46, 47 montés chacun sur une tige 37 et incorporant chacun un système de cliquet, lesdits corps étant reliés par un axe transversal 48 de section polygonale.

Cette partie mobile est conçue pour être associée à une vis pédiculaire 49 dotée d'une tête 50 présentant une gorge 51 de section polygonale conjuguée de celle de l'axe 48 et un filetage externe 52 en partie supérieure, et d'un capuchon 53 apte à venir se visser sur ladite tête après mise en place de la vis sur l'axe 48.

Le dispositif d'ostéosynthèse représenté à la figure 11 permet quant à lui de réaliser un montage en cadre.

Il comprend deux dispositifs d'ostéosynthèse conformes à ceux décrits en référence à la figure 9 dont les tiges 37 sont montées en parallèle et sont reliées par un dispositif d'ostéosynthèse conforme à celui décrit en référence aux figures 1 à 4 dont les crochets 4, 7 coopèrent avec lesdites tiges.

En dernier lieu, la figure 12 représente une variante d'un système de cliquet conforme à l'invention.

Selon cette variante, le corps 54 de la partie mobile du dispositif d'ostéosynthèse comporte un alésage 55 débouchant respectivement dans le fond du logement 56 du système de cliquet et au droit d'une des faces externes dudit corps, et présentant une section inférieure à celle dudit logement.

Le système de cliquet comporte quant à lui, un pion 57 doté d'une face frontale présentant en saillie une dent asymétrique 58, prolongé à l'opposé de ladite face frontale, par un axe 59 autour duquel est monté un ressort 60, ledit axe étant adapté pour s'étendre dans l'alésage 55 et déboucher à l'extérieur du corps 54.

De plus, cet axe 59 est doté, au niveau de son extrémité débouchante, d'un organe de manoeuvre 61 présentant, en regard de la face externe du corps 54, une face frontale 61a chanfreinée apte à permettre d'amener le système de cliquet à reculer au moyen d'un outil de forme conjuguée.

Une telle variante a pour avantage de permettre d'inverser de façon très aisée le sens autorisé de déplacement du dispositif d'ostéosynthèse, par une simple rotation de 180 degrés du système de cliquet, une fois celui-ci reculé de façon à dégager la dent asymétrique des crans 62 de l'élément longiforme 63 de la partie fixe.

De ce fait, un même dispositif peut être utilisé, sans démontage, pour des ostéosynthèses distractives et compressives. En outre, une telle variante présente un intérêt notable lors d'interventions telles qu'une ostéotomie où les parties osseuses doivent être dans un premier temps écartées, puis dans un deuxième temps comprimées.

## Revendications

1. Dispositif d'ostéosynthèse implantable comprenant deux parties, dites fixe (1, 3, 4 ; 23, 24 ; 14-19 ; 29-31, 34 ; 37, 39, 40, 41) et mobile (5, 7 ; 26, 27 ; 21, 22 ; 35 ; 41, 44, 45 ; 46-49), mobiles relativement l'une par rapport à l'autre et dotées de moyens d'association (2 ; 15 ; 25 ; 32 ; 38; 62), (10-13 ; 57-61) aptes à permettre, d'une part de leur conférer cette faculté de mobilité relative, et d'autre part, de les bloquer relativement en translation de façon à obtenir un effet de bridage d'un os :
- la partie fixe comportant un élément longiforme (1 ; 14 ; 23 ; 29-31 ; 37; 63),
- la partie mobile comportant un corps (5 ; 22 ; 26 ; 35 ; 39 ; 46, 47) formant un manchon (6) apte à coulisser le long de l'élément longiforme,
- et les moyens d'association comportant des moyens de verrouillage unidirectionnel (2 ; 15 ; 25 ; 32 ; 38; 62), (10-13 ; 57-61) aptes à autoriser le déplacement relatif desdites parties selon un seul sens de déplacement adapté pour permettre d'obtenir l'effet de bridage, et à verrouiller en translation ces parties fixe et mobile selon l'autre sens de déplacement,
- lesdits moyens de verrouillage unidirectionnel comportant d'une part, une pluralité de crans parallèles (2 ; 15 ; 25 ; 32 ; 38; 62) ménagés sur l'élément longiforme (1 ; 14 ; 23 ; 29-31 ; 37; 63) (de la partie fixe, et, d'autre part, un système de cliquet (10-13 ; 57-61) incorporé dans le corps (5 ; 22 ; 26 ; 35 ; 39 ; 46, 47) de la partie mobile, et des moyens élastiques (13 ; 60) adaptés pour venir se loger dans les crans (2 ; 15 ; 25 ; 32 ; 38; 62) de l'élément longiforme,
ledit dispositif d'ostéosynthèse étant caractérisé en ce que le système de cliquet comprend une dent asymétrique (12 ; 58) associée aux moyens élastiques (13 ; 60) de facon à pénétrer dans les crans (2 ; 15 ; 25 ; 32 ; 38; 62) de l'élément longiforme avec un jeu apte à permettre audit système de cliquet de basculer par rapport audit corps de façon à obtenir un effet de coin assurant un auto-blocage.

2. Dispositif d'ostéosynthèse selon la revendication 1, caractérisé en ce que la dent asymétrique (12 ; 58) s'étend en saillie par rapport à une des faces (11), dite supérieure, d'un pion d'encliquetage (10 ; 57) et comporte une face frontale inclinée (12a) apte à permettre le déplacement de la partie mobile selon un sens de déplacement, et une face frontale opposée (12b) de blocage en translation de ladite partie mobile, la portion (11a) de la face supérieure (11) du pion d'encliquetage (10 ; 57) jouxtant la face inclinée (12a) de la dent asymétrique (12 ; 58) s'étendant dans un plan inférieur par rapport à la portion (11b) de ladite face supérieure jouxtant la face de blocage (12b) de ladite dent asymétrique.

3. Dispositif d'ostéosynthèse selon l'une des revendications 1 ou 2, caractérisé en ce que le corps (5 ; 22 ; 26 ; 35 ; 39 ; 46, 47) de la partie mobile comporte un logement (9 ; 56) débouchant radialement dans le manchon (6) apte à loger le système de cliquet (10-13 ; 57-61).

4. Dispositif d'ostéosynthèse selon la revendication 3, caractérisé en ce que le corps (5 ; 22 ; 26 ; 35 ; 39 ; 46, 47) de la partie mobile est percé d'un alésage (8) ménagé transversalement par rapport au manchon (6) à partir d'une des faces externes dudit corps, de façon à former, à l'opposé de ladite face externe par rapport audit manchon, le logement (9 ; 56) au système de cliquet.

5. Dispositif d'ostéosynthèse selon l'une des revendications 3 ou 4, caractérisé en ce que :
- le corps (54) de la partie mobile est percé d'un alésage (55) ménagé transversalement par rapport au manchon à partir d'une des faces externes dudit corps, de façon à déboucher dans le fond du logement (56) du système de cliquet (57-61), ledit alésage présentant une section inférieure à celle audit logement,
- le système de cliquet comporte, à l'opposé de la dent asymétrique (58), un axe (59) de dimensions adaptées pour s'étendre dans l'alésage (55) et déboucher à l'extérieur du corps (54) de la partie mobile, ledit axe étant doté, au niveau de son extrémité débouchante, d'un organe de manoeuvre (61) apte à permettre de le faire pivoter sur lui-même.

6. Dispositif d'ostéosynthèse selon l'une des revendications précédentes, caractérisé en ce que :
- la partie fixe comporte un élément longiforme constitué d'une tige rectiligne (1), et des moyens de liaison comprenant un corps (3) solidaire d'une des extrémités de la tige rectiligne (1) et un organe de liaison (4) s'étendant dans le prolongement dudit corps et présentant la forme d'un crochet apte à venir coopérer avec un os,
- la partie mobile comprend un organe de liaison (7) s'étendant dans le prolongement du corps (5) de ladite partie mobile et présentant la forme d'un crochet similaire à celui de la partie fixe agencé pour s'étendre parallèlement à ce dernier.

7. Dispositif d'ostéosynthèse selon la revendication 6, caractérisé en ce que la tige rectiligne (1) comporte longitudinalement au moins une face plane, le manchon (6) ménagé dans le corps (5) de la partie mobile présentant une section conjuguée de celle de ladite tige rectiligne.

8. Dispositif d'ostéosynthèse selon l'une des revendications 1 à 5, caractérisé en ce que :
- la partie fixe comporte un élément longiforme (23) constitué d'une lame rigide rectiligne recourbée vers une de ses extrémités de façon à former un coude (24) de 180 degrés apte à venir coopérer avec un os,
- la partie mobile comprend un organe de liaison (27) porté par le corps (26) de ladite partie mobile et constitué d'une lame rigide courbe formant un coude de 180 degrés conjugué de celui de la lame rigide (23) de la partie fixe, agencé pour s'étendre parallèlement à ce dernier, ledit coude étant décalé latéralement par rapport à l'axe longitudinal de l'élément longiforme (23) de la partie fixe.

9. Dispositif d'ostéosynthèse selon l'une des revendications 1 à 5, caractérisé en ce que :
- la partie fixe comporte un élément longiforme constitué d'une broche cylindrique (14) dotée d'une extrémité en forme de pointe (14a) et comprenant une rainure annulaire (16) à proximité de ladite extrémité, et un organe de liaison présentant la forme d'une griffe (17) dotée d'un corps (18) comportant une gorge transversale (19) apte à venir se loger dans la rainure annulaire (16) de la broche (14),
- la partie mobile comprend un organe de liaison (21) s'étendant dans le prolongement du corps (22) de ladite partie mobile et présentant la forme d'une griffe similaire à celle de la partie fixe agencée pour s'étendre parallèlement à cette dernière.

10. Dispositif d'ostéosynthèse selon la revendication 9, caractérisé en ce que la gorge transversale (19) du corps (18) de la griffe (17) de la partie fixe présente longitudinalement un lamage (20) de section adaptée pour venir coiffer partiellement l'extrémité (14a) en forme de pointe de la broche (14) jouxtant la rainure annulaire (16) de cette dernière.

11. Dispositif d'ostéosynthèse selon l'une des revendications 1 à 5, caractérisé en ce que :
- la partie fixe comporte un élément longiforme constitué d'une lame rigide (28) présentant deux tronçons d'extrémité (29, 30) rectilignes et parallèles reliés par un tronçon médian (31) incliné par rapport à l'axe longitudinal de ces derniers, un desdits tronçons d'extrémité (30) présentant un orifice apte à loger une tête de vis (34), et l'autre tronçon d'extrémité (29) étant doté de moyens de verrouillage (32),
- l'organe mobile comporte au moins un organe de liaison (35) apte à pénétrer dans un os.

12. Dispositif d'ostéosynthèse selon l'une des revendications 1 à 5, caractérisé en ce que :
- la partie fixe comporte un élément longiforme constitué d'une tige rectiligne (37), et des moyens de liaison comprenant un corps (39) monté sur ledit élément longiforme prolongé par un axe (40) de section polygonale, et un organe de liaison consistant en une vis pédiculaire (41) dotée d'une tête (42) percée d'une lumière (43) de section polygonale conjuguée de celle de l'axe (40) du corps (39),
- la partie mobile comporte des moyens de liaison comprenant un axe (45) de section polygonale s'étendant dans le prolongement du corps (44) de ladite partie mobile, parallèlement par rapport à l'axe (40) des moyens de liaison de la partie fixe, et un organe de liaison consistant en une vis pédiculaire (41) dotée d'une tête (42) percée d'une lumière (43) de section polygonale conjuguée de celle dudit axe.

13. Dispositif d'ostéosynthèse selon la revendication 12, caractérisé en ce qu'il comprend :
- un ensemble fixe composé de deux parties fixes comportant chacune un élément longiforme constitué d'une tige rectiligne (37), et des moyens de liaison comprenant un corps (39) monté sur ledit élément longiforme, vers une des extrémités de ce dernier, prolongé par un axe (40) de section polygonale, et un organe de liaison consistant en une vis pédiculaire (41) dotée d'une tête (42) percée d'une lumière (43) de section polygonale conjuguée de celle de l'axe (40) du corps (39),
- un ensemble mobile intermédiaire de liaison des éléments longiformes (37) de l'ensemble fixe au droit d'un tronçon de ces derniers opposé à l'extrémité portant les moyens de liaison (39-41), adapté pour que lesdits éléments longiformes s'étendent parallèlement en étant partiellement décalés longitudinalement, ledit ensemble mobile intermédiaire comprenant une partie mobile (46, 47) montée sur chaque élément longiforme (37), et des moyens de liaison comportant, d'une part, un axe (48) de section polygonale reliant les corps (46, 47) desdites parties mobiles et s'étendant orthogonalement par rapport aux éléments longiformes (37) et, d'autre part, un organe de liaison consistant en une vis pédiculaire (49) dotée d'une tête (50) présentant une gorge (51) de section polygonale conjuguée de celle dudit axe et un filetage externe (52) en partie supérieure, et en un capuchon (53) apte à venir se visser sur la partie supérieure de ladite tête.

14. Dispositif d'ostéosynthèse, caractérisé en ce qu'il comprend en combinaison deux dispositifs d'ostéosynthèse conformes à la revendication 12, disposés parallèlement, et un dispositif d'ostéosynthèse conforme à l'une des revendications 7 ou 9 disposé de façon à relier les éléments longiformes (37) des deux dispositifs d'ostéosynthèse, et à former un cadre avec ces derniers.

15. Dispositif d'ostéosynthèse selon l'une des revendications 12 à 14, caractérisé en ce que le corps (39) des moyens de liaison (39-41) de chaque partie fixe comporte des moyens (10-13 ; 57-61) de verrouillage unidirectionnel aptes à permettre de le déplacer dans un seul sens de déplacement relativement à l'élément longiforme (37) correspondant.

## Patentansprüche

1. Einpflanzbare Vorrichtung für Osteosynthese mit zwei Teilen, sogenannt festen (1, 3, 4 ; 23, 24 ; 14-19 ; 29-31, 34 ; 37, 39, 40, 41) und beweglichen (5, 7 ; 26, 27 ; 21, 22 ; 35 ; 41, 44, 45 ; 46-49) Teilen, die beweglich relativ zueinander sind, und mit Schaltungsmitteln (2 ; 15 ; 25 ; 32 ; 38 ; 62), (10-13 ; 57-61) versehen sind, die sich dafür eignen, einerseits diese relative Bewegungsfähigkeit ihnen zu verleihen, und anderseits ihre relative Verschiebung zu blockieren, so daß ein Spanneffekt zum Spannen eines Knochens erreicht ist :
- wobei der feste Teil ein langgestrecktes Element (1 ; 14 ; 23 ; 29-31 ; 37 ; 63) aufweist,
- wobei der bewegliche Teil einen Körper (5 ; 22 ; 26 ; 35 ; 39 ; 46, 47) aufweist, der eine entlang dem langgestreckten Element gleitende Muffe (6) bildet,
- und wobei die Schaltungsmittel Verriegelungsmittel (2 ; 15 ; 25 ; 32 ; 38 ; 62), (10-13 ; 57-61) für die Verriegelung in einer einzigen Richtung aufweisen, die sich dafür eignen, die relative zum Erreichen des Spanneffekts taugliche Bewegung der genannten Teile in einer einzigen Richtung zu erlauben, und die Verschiebung dieser festen und beweglichen Teile in der anderen Bewegungsrichtung zu verriegeln,
- wobei die genannte Verriegelungsmittel für die Verriegelung in einer einzigen Richtung einerseits eine Vielzahl von parallelen Rasten (2 ; 15 ; 25 ; 32 ; 38 ; 62) im langgestreckten Element (1 ; 14 ; 23 ; 29-31 ; 37 ; 63) des festen Teils, und anderseits ein Klinkensystem (10-13 ; 57-61), eingebaut im Körper (5 ; 22 ; 26 ; 35 ; 39 ; 46, 47) des beweglichen Teils, und elastische Mittel (13 ; 60) aufweisen, die sich dafür eignen, sich in den Rasten (2 ; 15 ; 25 ; 32 ; 38 ; 62) des langgestreckten Elements einzufügen,
wobei die Vorrichtung für Osteosynthese dadurch gekennzeichnet ist, daß das Klinkensystem einen asymmetrischen den elastischen Mitteln (13 ; 60) zugehörigen Zahn (12 ; 58) umfaßt, so daß er sich in die Rasten (2 ; 15 ; 25 ; 32 ; 38 ; 62) des langgestreckten Elements mit einem Spiel einfügt, der die Umschaltung des genannten Klinkensystems bezüglich dem genannten Körper erlaubt, so daß ein Keileffekt zur Selbstblockierung erreicht ist.

2. Vorrichtung für Osteosynthese nach Anspruch 1, dadurch gekennzeichnet, daß der asymmetrische Zahn (12 ; 58) sich von einer der Flächen (11), sogenannter Oberfläche, eines Verklinkungsnockens (10 ; 57) vorspringend erstreckt, und eine geneigte Stirnfläche (12a), tauglich zum Erlauben der Bewegung des beweglichen. Teils in einer Bewegungsrichtung, und eine gegenüberliegende Stirnfläche (12b) für die Verschiebungsblockierung des genannten beweglichen Teils umfaßt, wobei der Anteil (11a) der Oberfläche (11) des Verklinkungsnockens (10 ; 57), angrenzend an die geneigte Fläche (12a) des asymmetrischen Zahns (12 ; 58), in einer unteren Ebene bezüglich dem Anteil (11b) der genannten Oberfläche, angrenzend an die Blockierungsfläche (12b) des genannten asymmetrischen Zahns, sich erstreckt.

3. Vorrichtung für Osteosynthese nach einem der Ansprüche 1 oder 2, dadurch gekennzeichnet, daß der Körper (5 ; 22 ; 26 ; 35 ; 39 ; 46 ; 47) des beweglichen Teils eine Aufnahme (9 ; 56) umfaßt, die radial in die Muffe (6) mündet, welche zur Aufnahme des Klinkensystems (10-13 ; 57-61) tauglich ist.

4. Vorrichtung für Osteosynthese nach Anspruch 3, dadurch gekennzeichnet, daß eine Bohrung (8) im Körper (5 ; 22 ; 26 ; 35 ; 39 ; 46, 47) des bewegliches Teils quer zur Muffe (6) ausgehend von einer der externen Flächen des genannten Körpers durchgebohrt ist, so daß die Aufnahme (9 ; 56) des Klinkensystems auf der gegenüberliegenden Seite der genannten externen Fläche bezüglich der genannten Muffe gebildet ist.

5. Vorrichtung für Osteosynthese nach einem der Ansprüche 3 oder 4, dadurch gekennzeichnet,
- daß eine Bohrung (55) im Körper (54) des beweglichen Teils quer zur Muffe ausgehend von einer der externen Flächen des genannten Körpers durchgebohrt ist, so daß sie in den Grund der Aufnahme (56) des Klinkensystems (57-61) mündet, wobei der Querschnitt der genannten Bohrung kleiner als der Querschnitt der genannten Aufnahme ist,
- daß das Klinkensystem auf der gegenüberliegenden Seite des asymmetrischen Zahns (58) eine Achse (59) mit Dimensionen umfaßt, die sich dafür eignen, sich in der Bohrung (55) zu erstrecken, und außerhalb des Körpers (54) des beweglichen Teils zu münden, wobei die genannte Achse an ihres mündende Ende mit einem Steuerorgan (61) versehen ist, die sich dafür eignet, deren Umdrehung auf sich zu erlauben.

6. Vorrichtung für Osteosynthese nach einem der vorhergehenden Ansprüche, dadurch gekennzeichnet,
- daß der feste Teil ein langgestrecktes Element, bestehend aus einer geradlinigen Stange (1), und Verbindungsmittel mit einem Körper (3) aus einem Stück mit einem der Enden der geradlinigen Stange (1) und mit einem Verbindungsorgan (4) umfaßt, welches Verbindungsorgan sich in der Verlängerung des genannten Körpers erstreckt und in Form von einem Haken ist, der sich dafür eignet, mit einem Knochen zusammenzuarbeiten,
- daß der bewegliche Teil ein Verbindungsorgan (7) umfaßt, das sich in der Verlängerung des Körpers (5) des genannten beweglichen Teils erstreckt, und in Form von einem Haken ist, der ähnlich wie der Haken des festen Teils ist, und so angeordnet ist, daß er sich parallel zum letzterer erstreckt.

7. Vorrichtung für Osteosynthese nach Anspruch 6, dadurch gekennzeichnet, daß die geradlinige Stange (1) in der Längsrichtung mindestens eine ebene Fläche umfaßt, wobei die Muffe (6) im Körper (5) des beweglichen Teils einen Querschnitt ausweist, der dem Querschnitt der genannten geradlinigen Stange entspricht.

8. Vorrichtung für Osteosynthese nach einem der Ansprüche 1 bis 5, dadurch gekennzeichnet,
- daß der feste Teil ein langgestrecktes Element (23) aus einem starren geradlinigen Streifen umfaßt, welcher Streifen auf der Seite eines ihrer Enden gebogen ist, so daß er eine 180° Biegung (24) bildet, die sich dafür eignet, mit einem Knochen zusammenzuarbeiten,
- daß der bewegliche Teil ein Verbindungsorgan (27) umfaßt, das durch den Körper (26) des genannten beweglichen Teils gehalten wird, und aus einem starren gebogenen Streifen besteht, der eine 180° Biegung bildet, die der Biegung des starren Streifens (23) des festen Teils entspricht, und so angeordnet ist, daß sie sich parallel zur letztere zu erstrecken, wobei die Biegung bezüglich der Längsachse des langgestreckten Elements (23) des festen Teils seitlich versetzt ist.

9. Vorrichtung für Osteosynthese nach einem der Ansprüche 1 bis 5, dadurch gekennzeichnet,
- daß der feste Teil ein langgestrecktes Element aus einem zylindrischen Stift (14) mit einem spitzenförmigen Ende (14a) und mit einer Ringnut (16) in der Nähe des genannten Endes, und ein Verbindungsorgan in Form von einer Klaue (17) mit einem Körper (18) umfaßt, der eine quere Vertiefung (19) aufweist, die sich dafür eignet, sich in die Ringnut (16) des Stifts (14) einzufügen,
- daß der bewegliche Teil ein Verbindungsorgan (21) umfaßt, der sich in der Verlängerung des Körpers (22) des genannten beweglichen Teils erstreckt, und in Form von einer Klaue ist, die ähnlich wie die Klaue des festen Teils ist, die so angeordnet ist, daß sie sich parallel zur letztere erstreckt.

10. Vorrichtung für Osteosynthese nach Anspruch 9, dadurch gekennzeichnet, daß die quere Vertiefung (19) des Körpers (18) der Klaue (17) des festen Teils in der Längsrichtung eine Senkung (20) aufweist, deren Querschnitt zum teilweisen Überdecken des spitzenförmigen Endes (14a) des an der Ringnut (16) der letztere angrenzenden Stifts angepaßt ist.

11. Vorrichtung für Osteosynthese nach einem der Ansprüche 1 bis 5, dadurch gekennzeichnet,
- daß der feste Teil ein langgestrecktes Element aus einem starren Streifen (28) mit zwei geradlinigen parallelen Endabschnitten (29, 30) umfaßt, die durch einem mittleren bezüglich der Längsachse der letztere geneigten Abschnitt (31) verbunden sind, wobei der eine genannte Endabschnitt (30) eine zur Aufnahme eines Schraubenkopfs (34) taugliche Öffnung aufweist, und der andere Endabschnitt (29) mit Verriegelungsmitteln (32) versehen ist,
- daß das bewegliche Organ mindestens ein zum Durchdringen in einen Knochen taugliches Verbindungsorgan (35) aufweist.

12. Vorrichtung für Osteosynthese nach einem der Ansprüche 1 bis 5, dadurch gekennzeichnet,
- daß das feste Teil ein langgestrecktes Element aus einer geradlinigen Stange (37), und Verbindungsmitteln mit einem Körper (39), der auf dem genannten langgestreckten Element, verlängert von einer Achse (40) mit vieleckigen Querschnitt, montiert ist, und mit einem Verbindungsorgan aus einem Schraubenstengel (41) mit einem Kopf (42) umfaßt, in welchem Kopf eine Durchgangsöffnung (43) mit vieleckigem Querschnitt, entsprechend dem Querschnitt der Achse (40) des Körpers (39), durchgebohrt ist,
- daß der bewegliche Teil Verbindungsmittel mit einer Achse (45) mit vieleckigem Querschnitt, die sich in der Verlängerung des Körpers (44) des genannten beweglichen Teils parallel zur Achse (40) der Verbindungsmittel des festen Teils erstreckt, und ein Verbindungsorgan aus einem Schraubenstengel (41) mit einem Kopf (42) umfaßt, in welchem Kopf eine Durchgangsöffnung mit vieleckigem Querschnitt, entsprechend dem Querschnitt der genannten Achse, durchgebohrt ist.

13. Vorrichtung für Osteosynthese nach Anspruch 12, dadurch gekennzeichnet, daß sie umfaßt :
- eine feste Baugruppe aus zwei festen Teilen jeweils mit einem langgestreckten Element aus einer geradlinigen Stange (37) und mit Verbindungsmitteln, die einen Körper (39), montiert auf dem genannten langgestreckten Element, verlängert auf der Seite eines seiner Enden von einer Achse (40) mit vieleckigem Querschnitt, und ein Verbindungsorgan aus einem Schraubenstengel (41) mit einem Kopf (42) umfaßt, in welchem Kopf eine Durchgangsöffnung (43) mit vieleckigem Querschnitt, entsprechend dem Querschnitt der Achse (40) des Körpers (39), durchgebohrt ist,
- eine zwischenliegende bewegliche Baugruppe für die Verbindung der langgestreckten Elemente (37) der festen Baugruppe in der Höhe eines Teilstücks dieser Elemente, das auf der gegenüberliegenden Seite bezüglich dem die Verbindungsmittel (39-41) haltenden Ende steht, und so angepaßt ist, daß die genannten langgestreckten Elemente sich mit einer teilweisen Längsversetzung parallel zueinander erstrecken, wobei die genannte zwischenliegende bewegliche Baugruppe einen beweglichen Teil (46, 47), montiert auf jedem langgestreckten Element (37), und Verbindungsmittel aufweist, die einerseits eine Achse (48) mit vieleckigem Querschnitt, welche die Körper (46, 47) der genannten beweglichen Teile verbindet, und sich senkrecht zu den langgestreckten Elementen (37) erstreckt, und anderseits ein Verbindungsorgan umfassen, das aus einem Schraubenstengel (49) mit einem Kopf (50), der mit einer Kehle (51) mit vieleckigem Querschnitt, entsprechend dem Querschnitt der genannte Achse, und mit einer Außengewinde (52) im Oberteil versehen ist, und aus einer Kappe (53), tauglich zum Schrauben auf den Oberteil des genannten Kopfs, besteht.

14. Vorrichtung für Osteosynthese, dadurch gekennzeichnet, daß sie in Zusammenstellung zwei parallel zueinander angeordnete Vorrichtungen für Osteosynthese gemäß dem Anspruch 12, und eine Vorrichtung für Osteosynthese gemäß einem der Ansprüche 7 oder 9 umfaßt, welche so angeordnet ist, daß sie die langgestreckten Elemente (37) der beiden Vorrichtungen für Osteosynthese verbindet, und mit diesen einen Rahmen bildet.

15. Vorrichtung für Osteosynthese nach einem der Ansprüche 12 bis 14, dadurch gekennzeichnet, daß der Körper (39) der Verbindungsmittel (39-41) jedes festen Teils Verriegelungsmittel (10-13 ; 57-61) für die Verriegelung in einer einzigen Richtung umfaßt, welche Verriegelungsmittel sich dafür eignen, seine Bewegung in einer einzigen Richtung bezüglich dem entsprechenden langgestreckten Element (37) zu erlauben.

## Claims

1. Implantable osteosynthesis device comprising two parts, called fixed (1, 3, 4; 23, 24; 14-19; 29-31, 34; 37, 39, 40, 41) and movable (5, 7; 26, 27; 21, 22; 35; 41, 44, 45; 46-49), one movable relative to the other and fitted with coupling means (2; 15; 25; 32; 38; 62), (10-13; 57-61) capable of making it possible, on the one hand, to confer on them this faculty of relative mobility and, on the other, of blocking them relatively in translation so as to obtain a clamping effect on the bone:
- the fixed part comprising an elongated element (1; 14; 23; 29-31; 37; 63),
- the movable part comprising a body (5; 22; 26; 35; 39; 46, 47) which forms a sleeve (6) capable of sliding along the elongated element,
- and the coupling means comprising one-way locking means (2; 15; 25; 32; 38; 62), (10-13; 57-61) capable of allowing the relative displacement of said parts in only one direction adapted to produce the clamping effect and to lock in translation these fixed and movable parts in the other direction,
- the said one-way locking means comprising, on the one hand, a number of parallel notches (2; 15; 25; 32; 38; 62) arranged along the elongated element (1; 14; 23; 29-31; 37; 63) of the fixed part and, on the other, a ratchet system (10-13; 57-61) incorporated in the body (5; 22; 26; 35; 39; 46, 47) of the movable part, and elastic means (13; 60) adapted to be accommodated in the notches (2; 15; 25; 32; 38; 62) of the elongated element,
the said osteosynthesis device being characterized in that the ratchet system comprises an asymmetric tooth (12; 58) combined with elastic means (13; 60) so as to penetrate into the notches (2, 15; 25; 32; 38; 62) of the elongated element with a clearance capable of enabling the said ratchet system to tilt with respect to said body so as to produce a wedge effect ensuring self-locking.

2. Osteosynthesis device according to Claim 1, characterized in that the asymmetric tooth (12; 58) extends in projection with respect to the so-called upper face (11) of a pawl (10; 57) and has an inclined front face (12a) capable of making possible the displacement of the movable part in one direction and an opposite front face (12b) for blocking translation of said movable part, the portion (11a) of the upper face (11) of the pawl (10; 57) adjacent to the inclined face (12a) of the asymmetric tooth (12; 58) extending at a lower level than the portion (11b) of said upper face adjacent to the blocking face (12b) of said asymmetric tooth.

3. Osteosynthesis device according to one of the Claims 1 or 2, characterized in that the body (5; 22; 26; 35; 39; 46, 47) of the movable part comprises a housing (9; 56) opening radially into the sleeve (6) capable of accommodating the ratchet system (10-13; 57-61).

4. Osteosynthesis device according to Claim 3, characterized in that a bore (8) arranged transversally with respect to the sleeve (6) is drilled through the body (5; 22; 26; 35; 39; 46, 47) of the movable part from one of the external faces of said body so as to form opposite said external face with respect to the sleeve the housing (9; 56) of the ratchet system.

5. Osteosynthesis device according to one of the Claims 3 or 4, characterized in that:
- a bore (55) arranged transversally with respect to the sleeve is drilled through the body (54) of the movable part from one of the external faces of said body so as to open into the bottom of the housing (56) of the ratchet system (57-61), said bore having a cross-section smaller than that of the housing,
- the ratchet system comprises opposite to the asymmetric tooth (58), an axis (59) of dimensio ns adapted for extension into the bore (55) and emergence outside of the body (54) of the movable part, said axis being fitted at its open end with a control device (61) capable of making it possible to swivel on itself.

6. Osteosynthesis device according to one of the preceding Claims, characterized in that:
- the fixed part comprises an elongated element constituted of a rectilinear bar (1) and coupling means comprising a body (3) forming a piece with one end of the rectilinear bar (1) and an attachment piece (4) extending in the prolongation of said body and possessing the form of a hook capable of being clamped to a bone,
- the movable part comprises an attachment piece (7) extending in the prolongation of the body (5) of said movable part and possessing the form of a hook similar to that of the fixed part arranged to extend in parallel with the latter.

7. Osteosynthesis device according to Claim 6, characterized in that the rectilinear bar (1) comprises longitudinally at least one flat face, the sleeve (6) arranged in the body (5) of the movable part having a cross-section coupled to that of said rectilinear bar.

8. Osteosynthesis device according to one of the Claims 1 to 5, characterized in that:
- the fixed part comprises an elongated element (23) constituted of a rectilinear rigid blade curved at one of its ends so as to form a bend (24) of 180 degrees capable of being clamped to a bone,
- the movable part comprises an attachment device (27) borne by the body (26) of said movable part and constituted of a curved rigid blade forming a bend of 180 degrees coupled to that of the rigid blade (23) of the fixed part, arranged so as to extend in parallel with this latter, said bend being laterally displaced with respect to the longitudinal axis of the elongated element (23) of the fixed part.

9. Osteosynthesis device according to one of the Claims 1 to 5, characterized in that:
- the fixed part comprises an elongated element constituted of a cylindrical spindle (14) equipped with a pointed end (14a) and comprising an annular bearing (16) close to said end and an attachment piece having the form of a claw (17) fitted with a body (18) comprising a transverse groove (19) capable of accommodating the annular bearing (16) of the spindle (14),
- the movable part comprises an attachment piece (21) extending in the prolongation of the body (22) of said movable part and having the form of a claw similar to that of the fixed part arranged to extend in parallel with this latter.

10. Osteosynthesis device according to Claim 9, characterized in that the transverse groove (19) of the body (18) of the clamp (17) of the fixed part possesses longitudinally a counterbore (20) of cross-section adapted to partially cap the pointed end (14a) of the spindle (14) adjacent to the annular bearing (16) of this latter.

11. Osteosynthesis device according to one of the Claims 1 to 5, characterized in that:
- the fixed part comprises an elongated element constituted of a rigid blade (28) exhibiting two parallel and rectilinear end sections (29, 30) connected by a central section (31) inclined with respect to the longitudinal axis of these latter, one of said end sections (30) possessing an aperture capable of accommodating a screw head (34) and the other end section (29) being equipped with locking means (32),
- the movable part comprises at least one attachment piece (35) capable of penetrating a bone.

12. Osteosynthesis device according to one of the Claims 1 to 5, characterized in that:
- the fixed part comprises an elongated element constituted of a rectilinear bar (37), and linking means comprising a body (39) mounted on said elongated element prolonged by an axis (40) of polygonal cross-section, and an attachment piece consisting of a pedicular screw (41) fitted with a head (42) through which is drilled a slot (43) of polygonal cross-section adapted to that of the axis (40) of the body (39),
- the movable part comprises linking means comprising an axis (45) of polygonal cross-section extending in the prolongatio n of the body (44) of said movable part, in parallel with the axis (40) of the linking means of the fixed part, and an attachment piece consisting of a pedicular screw (41) fitted with a head (42)) through which is drilled a slot (43) of polygonal cross-section adapted to that of said axis.

13. Osteosynthesis device according to Claim 12, characterized in that it comprises
- a fixed unit composed of two fixed parts each comprising an elongated element constituted of a rectilinear bar (37) and linking means comprising a body (39) mounted on said elongated element near one of the ends of this latter, extended by an axis (40) of polygonal cross-section and an attachment piece consisting of a pedicular screw (41) fitted with a head (42) through which is drilled a slot (43) of polygonal cross-section adapted to that of the axis (40) of the body (39),
- an intermediate movable unit linking the elongated elements (37) of the fixed unit in the region of a section of these latter opposite the end bearing the linking means (39-41), adapted so that said elongated elements extend in parallel while being partially displaced longitudinally, said intermediate movable unit comprising a movable part (46, 47) mounted on each elongated element (37) and linking means comprising, on the one hand, an axis (48) of polygonal cross-section connecting the bodies (46, 47) of said movable parts and extending at right angles with respect to the elongated elements (37) and, on the other, an attachment piece consisting of a pedicular screw (49) fitted with a head (50) possessing a groove (51) of polygonal cross-section adapted to that of said axis and an external thread (52) in the upper part, and a cap (53) capable of being screwed onto the upper part of said head.

14. Osteosynthesis device, characterized in that it comprises in combination two osteosynthesis devices in conformity with Claim 12, arranged in parallel, and an osteosynthesis device in conformity with one of the Claims 7 or 9 arranged so as to connect the elongated elements (37) of the two osteosynthesis devices, and to form a framework with these latter.

15. Osteosynthesis device according to one of the Claims 12 to 14, characterized in that the body (39) of the linking means (39-41) of each fixed part comprises one-way locking means (10-13; 57-61) capable of making it possible to displace it in only one direction relative to the corresponding elongated element (37). .
